# EUROPEAN PATENT APPLICATION

(11) **EP 1 930 441 A1**
(43) Date of publication of application: **11.06.2008**
(21) Application number: 06797245.5
(22) Date of filing: 01.09.2006
(51) Int. Cl.: C12P 41/00

(54) **METHOD FOR PRODUCTION OF OPTICALLY ACTIVE -HYDROXYCARBOXYLIC ACID**

(30) Priority: 02.09.2005 JP 2005254423
(71) Applicant: Daiichi Fine Chemical Co., Ltd., Takaoka-shi, Toyama 933-8511 (JP)
(72) Inventor: SAKAMOTO, Keiji, Takaoka-shi, Toyama 9338511 (JP); KITA, Shinji, Takaoka-shi, Toyama 9338511 (JP); MORII, Akihiro, Takaoka-shi, Toyama 9338511 (JP)
(74) Representative: Polypatent
(86) International application number: PCT/JP2006/317303
(87) International publication number: WO 2007/026860

(57) **Abstract**

An efficient method for producing an optically active α -hydroxycarboxylic acid represented by the following general formula (I) [A represents a residue of a 5- or 6-membered cyclic compound, * indicates a carbon atom in the S- or R-configuration, X represents hydrogen atom or an alkyl group having 1 to 4 carbon atoms], which comprises the step of treating a corresponding ester compound (not optically pure) with cell bodies or a culture, or a processed product or an extract thereof of a microorganism of the genus *Leifsonia,* genus *Cylindrocarpon,* genus *Verticillium,* or the like.

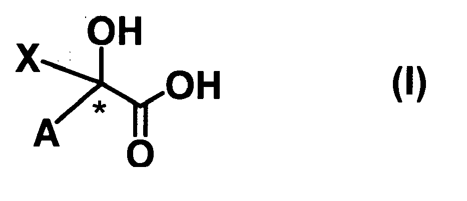

## Description

### Technical Field

The present invention relates to a method for producing an optically active α -hydroxycarboxylic acid or an optically active α -hydroxycarboxylic acid ester. More specifically, the present invention relates to a method for efficiently producing an optically active α-hydroxycarboxylic acid or an optically active α-hydroxycarboxylic acid ester from a racemic mixture of the α -hydroxycarboxylic acid ester by using a microorganism.

### Background Art

α -Hydroxycarboxylic acids and derivatives thereof are useful as intermediates for the manufacture of various kinds of medicaments or agricultural chemicals. Especially, since various biologically active compounds can be produced by using optically active α-hydroxycarboxylic acids or ester derivatives thereof having an asymmetric center at the α-position, various methods for efficiently producing optically active α-hydroxycarboxylic acids or ester derivatives thereof having a high optical purity has been developed. For example, it is known that clopidogrel (methyl (S)-2(2-chlorophenyl)-2-(4,5,6,7-tetrahydrothieno[3,2-c]-5-pyridyl)acetate, which is expected to be highly useful as a platelet coagulation inhibitor or an antithrombotic agent, can be efficiently produced from an optically active α -hydroxycarboxylic acid ester (Japanese Patent Unexamined Publication (KOKAI) No. 2001-519353). This method comprises the step of sulfonylating the α- hydroxyl group of methyl (R)-2-chloromandelate and reacting the resultant with 4,5,6,7-tetrahydrothieno[3,2-c]-5-pyridine.

As the method for producing an optically active α-hydroxycarboxylic acid or an ester thereof, the method of using optically active threo-1-(p-nitrophenyl)-2-amino-1,3-propanediol or optically active lysine is known, in which a diastereomeric salt thereof is prepared, and optical resolution of a racemic mixture of 2-chloromandelic acid is performed to prepare optically active 2-chloromandelic acid (Japanese Patent Unexamined Publication (KOKAI) No. 2004-530717). However, this method has a drawback of inevitable use of an expensive reagent as a resolving agent for the optical resolution.

A method of producing an optically active cyanohydrin (2-chloromandelonitrile) from 2-chlorobenzaldehyde and a cyanide donor (hydrogen cyanide and the like) by using hydroxynitrile lyase is proposed, in which optically active 2-chloromandelic acid is obtained from the optically active cyanohydrin by hydrolysis (Japanese Patent Unexamined Publication (KOKAI) No. 2004-57005). Also proposed is a method of performing asymmetric hydrolysis of cyanohydrin (2-chloromandelonitrile) obtained from 2-chlorobenzaldehyde and a cyanide donor (hydrogen cyanide and the like) to obtain optically active 2-chloromandelic acid (Japanese Patent Unexamined Publication (KOKAI) No. 4-99496). However, the cyanide donors (hydrogen cyanide and the like) used in these methods have a drawback that they are dangerous in handling.

Methods for obtaining an optically active mandelic acid derivative by using a microorganism having an ability to stereoselectively reduce α-carbonyl group of a phenylglyoxylic acid derivative are also proposed (Japanese Patent Unexamined Publication (KOKAI) Nos. 2003-199595 and 2004-49028). However, the α-keto acid (phenylglyoxylic acid derivative) used as the starting material is expensive, and a regeneration system of coenzymes is also required in these methods. Therefore, these methods suffer from a drawback of high production cost. Furthermore, also proposed is a method of subjecting a microorganism having an ability to stereoselectively oxidize a mandelic acid derivative to produce α-oxo compound to act on a racemic mixture of a mandelic acid derivative, and separating unreacted compounds to obtain the mandelic acid derivative with high optical purity (Japanese Patent Unexamined Publication (KOKAI) No. 6-165695). However, the separation of the α -oxo compound and the α-hydroxy compound in this method is complicated, and therefore the method suffers from a drawback that the separation itself is occasionally not achievable.

In addition, the methods described in Japanese Patent Unexamined Publication (KOKAI) Nos. 2-53497 and 2-156892 are also known as techniques concerning the method of producing optically active compounds characterized by use of enzymatic hydrolysis of an α-aryl-α-hydroxy acid ester. However, in the examples specifically disclosed in these patent documents, the methods are limited to those utilizing mandelic acid, and thus they have a problem of limited usefulness. Further, although a method of enzymatically hydrolyzing an α-aryl- α-hydroxy acid ester having various substituents is disclosed in Canadian Journal of Chemistry, 68 (2), p.314, 1990, the enzyme used is limited to carbonic anhydrase, and the optical purity of the product is about 40 to 50%ee, which means low selectivity, and therefore the method has a problem of low usefulness.
Patent document 1: Japanese Patent Unexamined Publication (KOKAI) No. 2001-519353
Patent document 2: Japanese Patent Unexamined Publication (KOKAI) No. 2004-530717
Patent document 3: Japanese Patent Unexamined Publication (KOKAI) No. 2004-57005
Patent document 4: Japanese Patent Unexamined Publication (KOKAI) No. 4-99496 Patent document 5: Japanese Patent Unexamined Publication (KOKAI) No. 2003-199595
Patent document 6: Japanese Patent Unexamined Publication (KOKAI) No. 2004-49028
Patent document 7: Japanese Patent Unexamined Publication (KOKAI) No. 6-165695 Patent document 8: Japanese Patent Unexamined Publication (KOKAI) No. 2-53497 Patent document 9: Japanese Patent Unexamined Publication (KOKAI) No. 2-156892 Non-patent document 1: Canadian Journal of Chemistry, 68 (2), p.314, 1990

### Disclosure of the Invention

### Object to be Achieved by the Invention

An object of the present invention is to provide an efficient method for producing an optically active α-hydroxycarboxylic acid or optically active α-hydroxycarboxylic acid ester. More specifically, the object of the present invention is to provide a method for efficiently producing an optically active α-hydroxycarboxylic acid or an optically active α-hydroxycarboxylic acid ester from a racemic mixture of the α-hydroxycarboxylic acid ester by using a microorganism.

### Means for Achieving the Object

The inventors of the present invention conducted various researches to achieve the aforementioned object, and as a result, they found that when a racemic mixture of an α -hydroxycarboxylic acid ester is hydrolyzed by using a microorganism belonging to a particular genus or a extract thereof, hydrolysis of the ester stereoselectively advanced, and an optically active α -hydroxycarboxylic acid or optically active hydroxycarboxylic acid ester was successfully produced with high efficiency by using the hydrolysis reaction. The present invention was achieved on the basis of the aforementioned finding.

The present invention thus provides a method for producing a compound represented by the general formula (I): 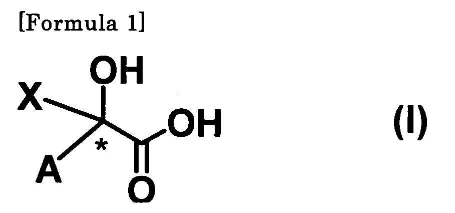 [wherein A represents a residue of a 5- or 6-membered cyclic compound, wherein the cyclic compound is selected from an aromatic compound, a partially saturated cyclic compound, or a saturated cyclic compound, and wherein said compound may have one or more heteroatoms as a ring-constituting atom, and may have a substituent on the ring (the substituent consists of one or two or more substituents selected from the group consisting of a halogen atom, an alkyl group having 1 to 4 carbon atoms which may have a substituent, an alkyloxy group having 1 to 4 carbon atoms which may have a substituent, a hydroxyl group which may be protected with a protective group, an amino group which may be protected with a protective group, and nitro group, and when two or more substituents exist, they may be the same or different, and they may bind to each other to form a ring), X represents hydrogen atom or an alkyl group having 1 to 4 carbon atoms, and * indicates a carbon atom in the S- or R-configuration], which comprises the step of treating a compound represented by following general formula (II): 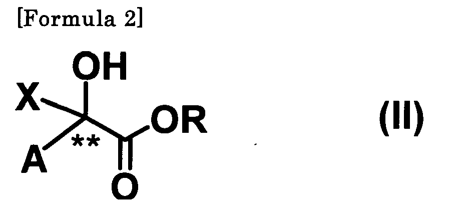 [wherein A and X have the same meanings as those defined above, and R represents an alkyl group having 1 to 4 carbon atoms (the alkyl group may be substituted with an aryl group), provided that the compound represented by the general formula (II) is not optically pure for the carbon atom indicated with **] with cell bodies or a culture, or a processed product or an extract thereof of a microorganism selected from the group consisting of microorganisms belonging to the genus *Leifsonia,* genus *Cylindrocarpon,* genus *Verticillium,* genus *Mycobacterium,* genus *Rhodococcus,* genus *Exophiala,* genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhinocladiella,* genus *Ramichloridium* or genus *Porphyrobacter.*

According to a preferred embodiment of the aforementioned invention, there is provided the aforementioned method, wherein A is chlorophenyl group, R is an alkyl group having 1 to 4 carbon atoms or benzyl group, and X is hydrogen atom. According to another preferred embodiment of the present invention, there is also provided the aforementioned method, which comprises the step of hydrolyzing the ester group of the compound represented by the general formula (II) wherein the carbon atom indicated with ** is in the R-configuration to obtain the compound represented by the general formula (I) wherein the carbon atom indicated with * is in the R-configuration. According to still another preferred embodiment of the present invention, there is also provided the aforementioned method, which comprises the step of hydrolyzing the ester group of the compound represented by the general formula (II) wherein the carbon atom indicated with ** is in the S-configuration to obtain the compound represented by the general formula (I) wherein the carbon atom indicated with * is in the S-configuration.

From another aspect, there is provided a method for producing a compound represented by the following general formula (III): 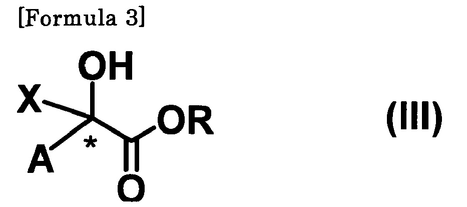 (wherein A, R, X and * have the same meanings as those defied above), which comprises the step of treating a compound represented by the aforementioned general formula (II) (wherein A, R, X, and ** have the same meanings as those defined above) with cell bodies or a culture, or a processed product or an extract thereof of a microorganism selected from the group consisting of microorganisms belonging to the genus *Leifsonia,* genus *Cylindrocarpon,* genus *Verticillium,* genus *Mycobacterium,* genus *Rhodococcus,* genus *Exophiala,* genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhinocladiella* group, genus *Ramichloridium* or genus *Porphyrobacter* to hydrolyze the ester group of the compound represented by the general formula (II) wherein the carbon atom indicated with ** is in the S- or R-configuration, and separating the unreacted ester compound which is optically pure and remains in a reaction mixture.
According to a preferred embodiment of the aforementioned invention, there is provided the aforementioned method, wherein A is chlorophenyl group, R is an alkyl group having 1 to 4 carbon atoms or benzyl group, and X is hydrogen atom.

### Best Mode for Carrying out the Invention

In the compounds represented by the general formula (I), A represents a residue of a 5- or 6-membered cyclic compound. The residue means a monovalent group obtained by eliminating one hydrogen binding to a ring-constituting atom of a cyclic compound. The cyclic compound may be any of an aromatic compound, a partially saturated cyclic compound, or a saturated cyclic compound, and may have one or more heteroatoms as a ring-constituting atom. Although type of the heteroatom is not particularly limited, nitrogen atom, oxygen atom, sulfur atom or the like can be used, for example, and when the ring contains two or more ring-constituting heteroatoms, they may be the same or different. Examples of the cyclic compound include, more specifically, benzene, 5- or 6-membered aromatic heterocyclic compounds (for example, furan, thiophene, pyridine, pyrimidine and the like), 5- or 6-membered aliphatic cyclic compounds (for example, cyclopentane, cyclohexane, cyclohexene and the like), 5- or 6-membered heterocyclic compounds (for example, pyrrolidine, piperidine, piperazine, morpholine, dihydrofuran, tetrahydrofuran and the like), and the like. As the cyclic compound, benzene is preferred among them.

The cyclic compound may have a substituent, and the substituent consists of one or two or more substituents selected from the group consisting of a halogen atom, an alkyl group having 1 to 4 carbon atoms which may have a substituent, an alkyloxy group having 1 to 4 carbon atoms which may have a substituent, a hydroxyl group which may be protected with a protective group, an amino group which may be protected with a protective group, and nitro group. When the cyclic compound has a substituent, number and substituting position of the substituent are not particularly limited. When the compound has two or more substituents, they may be the same or different. When the aforementioned alkyl group or alkyloxy group has a substituent, type, number and substituting position of the substituent are not particularly limited, and when the group has two or more substituents, they may be the same or different. Examples of the substituent of the aforementioned alkyl group or alkyloxy group include, for example, hydroxyl group, a halogen atom, amino group and the like, but not limited to these examples. When the cyclic compound has two or more substituents, those substituents may combine with each other to constitute a ring. In this case, the ring system may be aromatic, partially saturated or fully saturated. For example, examples include the compound wherein two alkyl groups bind together to form a carbon ring, and wherein one alkyloxy group and hydroxyl group bind together to form a ring system of an alkylenedioxy group and the like. However, the compounds are not limited to these examples.

Examples of the protective group include, for example, protective groups described in Protective Groups in Organic Chemistry (J.F.W. McOmie et al., Plenum Press) and Protective Groups in Organic Synthesis, 3rd Edition (Theodora W. Green, Peter G.M. Wuts, John Wily & Sons, Inc. (ISBN O-471-16019-9), April 1999), and specific examples include ether type protective groups such as methyl group, ethyl group, isopropyl group, t-butyl group, methoxymethyl group, benzyloxymethyl group, methoxyethoxymethyl group, methylthiomethyl group, phenylthiomethyl group, tetrahydropyranyl group, p-bromophenacyl group, allyl group and cyclohexyl group; benzyl type protective groups such as benzyl group, 2,6-dimethylbenzyl group, 4-methoxybenzyl group, 2,6-dichlorobenzyl group, 9-anthranylmethyl group, diphenylmethyl group, phenethyl group and triphenylmethyl group; silyl type protective groups such as trimethylsilyl group, triethylsilyl group, dimethylethylsilyl group and t-butyldimethylsilyl group; acyl type protective groups such as acetyl group, chloroacetyl group, trifluoroacetyl group and pivaloyl group; aroyl type protective groups such as benzoyl group, p-methylbenzoyl group, p-chlorobenzoyl group, o-chlorobenzoyl group and p-nitrobenzoyl group; carbonate type protective groups such as methoxycarbonyl group, ethoxycarbonyl group, t-butoxycarbonyl group, benzyloxycarbonyl group and p-methylbenzyloxycarbonyl group; phosphinate type protective groups such as dimethylphosphinyl group and diethylphosphinyl group; sulfonyl type protective groups such as methanesulfonyl group, ethanesulfonyl group, chloromethanesulfonyl group, chloroethanesulfonyl group, trichloromethanesulfonyl group, trifluoromethanesulfonyl group, benzenesulfonyl group, p-toluenesulfonyl group, o-nitrobenzenesulfonyl group, m-nitrobenzenesulfonyl group, p-nitrobenzenesulfonyl group, o-chlorobenzenesulfonyl group, m-chlorobenzenesulfonyl group and p-chlorobenzenesulfonyl group, and the like.

In the compounds represented by the general formula (I), (II) or (III), X represents an alkyl group having 1 to 4 carbon atoms. The alkyl group may be linear or branched.
In the compounds represented by the general formula (II), R represents an alkyl group having 1 to 4 carbon atoms. The alkyl group may be substituted with one or two or more aryl groups, and as the aryl group, phenyl group and the like are preferred. Examples of the alkyl group substituted with an aryl group such as phenyl group include benzyl group, benzhydryl group, phenethyl group, and the like.

The method of the present invention is that for producing a compound represented by the aforementioned general formula (I), and is characterized by comprising the step of treating a compound represented by the aforementioned general formula (II) with cell bodies or a culture, or a processed product or an extract thereof of a microorganism belonging to any one of the following genera. In the general formula (I), * indicates a carbon atom in the S- or R-configuration, and the compound represented by the general formula (I) is a substantially optically pure compound with reference to said asymmetric carbon. When the compound represented by the general formula (I) has another asymmetric carbon, the configuration thereof is not particularly limited. Further, in the compound represented by the general formula (II), ** indicates that the compound represented by the general formula (II) is not substantially optically pure with reference to said carbon atom. For example, a mixture of S-isomer and R-isomer at an arbitrary ratio, a racemate thereof and the like as for this carbon atom can be used. The microorganism used for the method of the present invention is that belonging to any one of the following genera: genus *Leifsonia,* genus *Cylindrocarpon,* genus *Verticillium,* genus *Mycobacterium,* genus *Rhodococcus,* genus *Exophiala,* genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhinocladiella,* genus *Ramichloridium* genus, and genus *Porphyrobacter.* More specifically, examples of the microorganism used for the method of the present invention include *Leifsonia aquatica, Cylindrocarpon* sp., *Verticillium leptobactrum, Mycobacterium smegmatis, Mycobacterium phlei, Mycobacterium vaccae, Rhodococcus equi, Rhodococcus fascians, Rhodococcus wratislaviensis, Exophiala jeanselmei, Exophiala dermatitidis, Rhodotorula aurantiaca, Bacillus cereus, Bacillus fusiformis, Brevundimonas diminuta, Pseudomonas aeruginosa, Rhizobium radiobacter, Aspergillus ochraceus, Aspergillus oryzae, Beauveria bassiana, Penicillium spin ulosum, Nocardia asteroides, Nocardia globerula, Gordonia bronchialis, Gordonia sputi, Gordonia rubripertincta, Rhodococcus* sp., *Rhodococcus rhodochrous, Rhodococcus erythropolis, Rhinocladiella ellisii, Rhinocladiella atrovirens, Ramichloridium anceps, Porphyrobacter sanguineus,* and the like. However, microorganisms are not limited to these examples.

An example of preferred embodiments of the aforementioned method includes the method comprising the step of hydrolyzing the ester group of the compound represented by the general formula (II) wherein the carbon atom indicated with ** is in the R-configuration to obtain the compound represented by the general formula (I) wherein the carbon atom indicated with * is in the R-configuration. In this embodiment, a microorganism belonging to any one of the following genera can be used: genus *Leifsonia,* genus *Cylindrocarpon,* genus *Verticillium,* genus *Mycobacterium,* genus *Rhodococcus,* genus *Exophiala* and genus *Rhinocladiella*. More specifically, examples of microorganisms belonging to these genera include the following microorganisms, but are not limited to these examples: *Leifsonia aquatica, Cylindrocarpon* sp., *Verticillium leptobactrum, Mycobacterium smegmatis, Mycobacterium phlei, Mycobacterium vaccae, Rhodococcus equi, Rhodococcus fascians, Rhodococcus wratislaviensis, Exophiala jeanselmei, Exophiala dermatitidis, Rhinocladiella ellisii,* and *Rhinocladiella atrovirens.*

Another example of preferred embodiments of the aforementioned method includes the method comprising the step of hydrolyzing the ester group of the compound represented by the general formula (II) wherein the carbon atom indicated with ** is in the S-configuration to obtain the compound represented by the general formula (I) wherein the carbon atom indicated with * is in the S-configuration. In this embodiment, a microorganism belonging to any one of the following genera can be used: genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhodococcus,* genus *Ramichloridium,* and genus *Porphyrobacter.* More specifically, examples of microorganisms belonging to these genera include the following microorganisms, but are not limited to these examples: *Rhodotorula aurantiaca, Bacillus cereus, Bacillus fusiformis, Brevundimonas diminuta, Pseudomonas aeruginosa, Rhizobium radiobacter, Aspergillus ochraceus, Aspergillus oryzae, Beauveria bassiana, Penicillium spinulosum, Nocardia asteroides, Nocardia globerula, Gordonia bronchialis, Gordonia sputi, Gordonia rubripertincta, Rhodococcus* sp., *Rhodococcus rhodochrous, Rhodococcus erythropolis, Ramichloridium anceps,* and *Porphyrobacter sanguineus.*

The method of the present invention provided from another aspect is a method for producing a compound represented by the aforementioned general formula (III), and is characterized by comprising the step of treating a compound represented by the aforementioned general formula (II) with cell bodies or a culture, or a processed product or an extract thereof of a microorganism belonging to any one of the following genera to hydrolyze the ester group of the compound represented by the general formula (II) wherein the carbon atom indicated with ** is in the S- or R-configuration and separating the unreacted ester compound which is optically pure and remains in a reaction mixture. Also in this method, a microorganism belonging to any one of the following genera can be used: genus *Leifsonia,* genus *Cylindrocarpon,* genus *Verticillium,* genus *Mycobacterium,* genus *Rhodococcus,* genus *Exophiala,* genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhinocladiella,* genus *Ramichloridium,* and genus *Porphyrobacter.*

In one of preferred embodiments of this method, the method comprises the step of hydrolyzing the ester group of the compound (II) wherein the carbon atom indicated with ** is in the R-configuration. In this preferred embodiment, a microorganism belonging to any one of the following genera can be used: genus *Leifsonia,* genus *Cylindrocarpon,* genus *Verticillium,* genus *Mycobacterium,* genus *Rhodococcus,* genus *Exophiala,* and genus *Rhinocladiella.* More specifically, examples of microorganisms belonging to these genera include the following microorganisms, but are not limited to these examples: *Leifsonia aquatica, Cylindrocarpon* sp., *Verticillium leptobactrum, Mycobacterium smegmatis, Mycobacterium phlei, Mycobacterium vaccae, Rhodococcus equi, Rhodococcus fascians, Rhodococcus wratislaviensis, Exophiala jeanselmei, Exophiala dermatitidis, Rhinocladiella ellisii,* and *Rhinocladiella atrovirens.*

In another preferred embodiment of this method, the method comprises the step of hydrolyzing the ester group of the compound (II) wherein the carbon atom indicated with ** is in the S-configuration. In this preferred embodiment, a microorganism belonging to any one of the following genera can be used: genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhodococcus,* genus *Ramichloridium* group, and genus *Porphyrobacter.* More specifically, examples of microorganisms belonging to these genera include the following microorganisms, but are not limited to these examples: *Rhodotorula aurantiaca, Bacillus cereus, Bacillus fusiformis, Brevundimonas diminuta, Pseudomonas aeruginosa, Rhizobium radiobacter, Aspergillus ochraceus, Aspergillus oryzae, Beauveria bassiana, Penicillium spinulosum, Nocardia asteroides, Nocardia globerula, Gordonia bronchialis, Gordonia sputi, Gordonia rubripertincta, Rhodococcus* sp., *Rhodococcus rhodochrous, Rhodococcus erythropolis, Ramichloridium anceps,* and *Porphyrobacter sanguineus.*

The aforementioned microorganisms are mentioned only by way of examples, and two of microbial strains belonging to the same genus may give converse stereoselectivities in the aforementioned hydrolysis reaction. Whether or not a compound represented by the general formula (I) as a resultant of the hydrolysis has a desired stereochemistry, or whether or not the compound has a converse stereochemistry of the desired steric configuration can be easily confirmed by those skilled in the art according to the methods specifically described in the examples of this specification, thereby the microbial strain used is easily identified to have which of the stereoselectivities. When a microorganism having the latter stereoselectivity is used, a compound of the general formula (I) having the desired configuration can be obtained by separating and purifying an unreacted starting compound from the reaction mixture, and then performing the hydrolysis reaction as explained above. Further, by using a microorganism having such stereoselectivity to hydrolyze only the ester compound represented by the general formula (III) having the converse of the desired stereochemistry, the ester compound of the general formula (III) having the desired stereochemistry can be obtained by separating and purifying the unreacted starting compound from the reaction mixture, and further performing the hydrolysis reaction.

A compound obtained by esterification of a compound of the general formula (I) having the converse of the desired stereochemistry, or a compound of the general formula (III) having the converse of the desired stereochemistry can be converted into a compound of the general formula (III) which is not optically pure in a conventional manner. For example, such a compound can be racemized by heating in a solution in the presence of a strongly basic substance. By subjecting a compound of the general formula (III) which is not optically pure and obtained as described above to a microbial reaction similar to that mentioned above, a compound having the converse of the desired stereochemistry can be recycled without discarding the compound.

The aforementioned microorganism used in the methods of the present invention may be any of wild strains, variant strains, and recombinant strains derived by a cell engineering technique such as cell fusion or a gene engineering technique such as DNA cloning and genetic manipulation. These microorganisms can be obtained from various culture collection organizations. For example, they can be obtained from Institute of Molecular and Cellular Biosciences, University of Tokyo (IAM), the independent administrative agency, National Institute of Technology and Evaluation (NBRC) (formerly Institute for Fermentation, Osaka (IFO)), the independent administrative agency, Institute of Physical and Chemical Research (JCM), and the like.

In the methods of the present invention, cell bodies of the aforementioned microorganisms can be used. Examples of the cell bodies include cell bodies collected from culture medium of the aforementioned microorganisms, cell bodies obtained by collecting the cell bodies from the culture medium and washing the cell bodies, cell bodies subjected to drying or acetone powder treatment, and the like, and any of these cell bodies can be preferably used. Further, the cell bodies can also be immobilized with an appropriate means and used. Immobilization can be attained by methods well known to those skilled in the art (for example, crosslinking, physical adsorption, entrapment and the like). Immobilization carrier may be any of those generally used, and examples include, for example, polysaccharides such as cellulose, agarose, dextran, κ-carrageenan, alginic acid, gelatin and cellulose acetate; natural polymers such as gluten; inorganic substances such as activated carbon, glass, white clay, kaolinite, alumina, silica gel, bentonite, hydroxyapatite and calcium phosphate; synthetic adsorbent materials such as polyacrylamide, polyvinyl acetate, polypropylene glycol and urethane, and the like. The cell bodies can also be used in the form of those encapsulated in microcapsules. The form for use of the cell bodies is not limited to those mentioned above, and it should be understood that any form can be appropriately selected from those available in this field and used by those skilled in the art.

In the methods of the present invention, culture of the aforementioned microorganisms may also be used. Examples of the culture include culture obtained by culturing the aforementioned microorganisms in a suitable medium. In the methods of the present invention, a processed product or an extract of the aforementioned microorganisms can also be used. Examples of the processed product include digest obtained by autolysis of the cell bodies or a culture suspended in a buffer if needed, disrupted cell bodies or a culture disrupted by using a physical means such as mortar, Dynomill, French press, supersonic wave and homogenizer, disrupted cell bodies or a culture by such methods in combination with an enzymatic means such as lysozyme, and the like. Examples of the extract include extracts of the cell bodies or a culture, or a processed product thereof obtained by extraction with water or an appropriate buffer as well as precipitates obtained from the foregoing extracts by salting out with ammonium sulfate, precipitates obtained from the foregoing extracts by precipitation with alcohol or the like, purification products of the foregoing extracts or precipitates obtained by gel filtration using Sephadex or the like, hydrophobic chromatography using a carrier having hydrophobic groups such as butyl group, octyl group and phenyl group, ion exchange chromatography using a carrier having diethylaminoethyl group or carboxymethyl group or the like, dye gel chromatography, electrophoresis, dialysis, ultrafiltration, affinity chromatography, high performance liquid chromatography, or the like, and the examples further include, for example, those containing an enzyme. The term "extract" used in this specification should construed in the widest sense thereof including enzyme solutions, isolated and/or purified enzymes, and the like. As the enzyme, specifically, lipase, α-amylase, acylase, and the like are preferably used. As these enzymes, purified enzymes derived from the aforementioned microorganisms (for example, commercially available enzymes) may also be used.

Conditions for the culture of the aforementioned microorganisms are not particularly limited, and can be suitably selected from ordinary culture conditions suitable for the culture of the microorganisms. Type of the medium is not also particularly limited, and a medium suitable for any one of bacteria, fungi and yeast can be suitably chosen. As the medium, a liquid medium containing a carbon source, a nitrogen source and other nutrients can usually be used. The carbon source of the medium is not particularly limited so long as a substance that can be utilized by the aforementioned microorganisms is chosen, and an arbitrary carbon source can be used. More specifically, examples of the carbon source include assimilable substances, and for example, saccharides such as glucose, fructose, sucrose, dextrin, starch and sorbitol, alcohols such as methanol, ethanol and glycerol, organic acids such as fumaric acid, citric acid, acetic acid and propionic acid and salts thereof, hydrocarbons such as paraffin, molasses, mixtures of these, and the like can be used.

The nitrogen source is not particularly limited so long as a substance that can be utilized by the aforementioned microorganisms is chosen, and an arbitrary nitrogen source can be used. More specifically, examples of the nitrogen source include assimilable substances, and for example, inorganic or organic nitrogen-containing compounds, for example, ammonium salts of inorganic acids such as ammonium chloride, ammonium sulfate, ammonium nitrate and ammonium phosphate, ammonium salts of organic acids such as ammonium fumarate and ammonium citrate, nitrates such as sodium nitrate and potassium nitrate, meat extract, yeast extract, malt extract, peptone, corn steep liquor, soybean protein hydrolysate, mixtures thereof, and the like can be used. Moreover, to the medium, nutrients used for usual culture, for example, inorganic substances such as potassium phosphate, iron sulfate, zinc sulfate, and manganese sulfate, trace element salts, vitamins, and the like may be optionally added. To the medium, a substance which induces activity of microorganisms, a buffering substance effective for pH maintenance of the medium, an antifoam, silicone, Adecanol, Pluronic, and the like may also be added, if needed.

Culture of the microorganisms can be performed under conditions suitable for growth of each microorganism, and such conditions can be suitably chosen by those skilled in the art. For example, the culture may be performed at pH 3 to 10, preferably pH 4 to 9, of the medium, and at a temperature of 0 to 50°C, preferably 20 to 40°C. Culture of the microorganisms can be performed under aerobic or anaerobic condition depending on the property of each microorganism. Although culture time is 1 to 300 hours, preferably 10 to 150 hours, it can be suitably determined for each microorganism.

In the methods of the present invention, conditions for treating the compound represented by the general formula (II) with cell bodies or a culture of the aforementioned microorganisms, or a processed product or an extract thereof are not particularly limited, and any conditions may be chosen so long as the aforementioned compound can fully contact with the cell bodies or the culture, or the processed product or the extract thereof, and the hydrolysis reaction of the ester moiety advances as a result. For example, cell bodies washed with a buffer or water or culture, or a processed product or an extract thereof may be mixed with a solution of a compound represented by the general formula (II). Although the aforementioned step can be performed in a homogeneous aqueous system or a two-phase system of a substantially water-insoluble or hardly water-soluble organic solvent and water, it is generally preferable to perform the step in a homogeneous aqueous system. As the solvent for forming the homogeneous aqueous system, water alone may be used as the solvent, or a mixture of a suitable water miscible organic solvent such as ethanol, methanol, dioxane and dimethyl sulfoxide and water may be used. A compound represented by the general formula (II) may be dissolved in the aforementioned organic solvent, and the resulting solution may be used by adding the solution to an aqueous solution or aqueous suspension containing cell bodies or a culture of the aforementioned microorganisms, or a processed product thereof or an extract thereof.

Further, the microorganism and the extract may be those obtained by subjecting the cell bodies or a culture to a heat treatment if needed, or those obtained by subjecting such heat-treated products to a single or at least two appropriate treatments. The heat treatment can be performed by an arbitrary method available in this field, and heat treatment conditions can be suitably determined by experiments or the like depending on a purpose. Temperature of the heat treatment is, for example, about 37°C or higher, preferably about 40 to 70°C, more preferably about 45 to 60°C. Although a period of time for the heat treatment can be suitably chosen depending on the treatment temperature, the period of time is, for example, about 5 minutes to about 24 hours, preferably about 30 minutes to 10 hours, more preferably about 1 to 5 hours. Typical heat treatment includes the step of heat treatment at a temperature of about 45°C, about 50°C, or about 55°C for about 2 to 4 hours, more preferably the step of heat treatment at a temperature of about 45 to 55°C for about 3 hours. By using heat-treated cell bodies or a culture, favorable results may be obtained for selectivity, conversion ratio, and the like.

Conditions of the treatment are not particularly limited, so long as conditions under which the asymmetric hydrolysis reaction of the ester advances are chosen. Although amount of the cell bodies for use as dry cell bodies or volume of extract or the like added in the case of using the extract or the like is not particularly limited, the amount is, for example, about 1/100 to 1000 times, preferably 1/10 to 100 times, based on the compound represented by the general formula (II). Concentration of the compound represented by the general formula (II) as the substrate is 0.01 to 20% by weight, preferably 0.1 to 10% by weight, to the total weight of the reaction system. Further, pH of the reaction mixture is 4 to 9, preferably 5 to 8, and the reaction temperature is 10 to 50°C, preferably 20 to 40°C. For stabilizing pH, a buffer can also be used. As the buffer, phosphate buffer, Tris buffer, acetate buffer, and the like can be used. Furthermore, for adjustment of pH, an acid or a base may be used to adjust pH. Although the reaction time is 1 to 200 hours, preferably 5 to 150 hours, it can be suitably chosen depending on each microorganism. If needed, the substrate and/or cell bodies or a culture of the microorganisms, or a processed product or an extract thereof may be added to the reaction system at one time, batchwise, or continuously. By continuously extracting the hydrolysate as a product, the reaction rate can also be increased.

An optically active α -hydroxycarboxylic acid represented by the general formula (I) or an optically active α -hydroxycarboxylic acid ester represented by the general formula (III) obtained by the reaction can be isolated and purified by conventional separation and purification means. For example, after separating cell bodies from the reaction mixture if needed, the culture can be purified by a usual purification method such as membrane separation, extraction with an organic solvent (for example, toluene, chloroform and the like), column chromatography, vacuum concentration, distillation, crystallization and recrystallization to obtain an optically active α -hydroxycarboxylic acid represented by the general formula (I) or an optically active α -hydroxycarboxylic acid ester represented by the general formula (III). Moreover, after completion of the reaction, a crude product can be obtained by, for example, extraction of the product from the reaction mixture with an organic solvent such as butyl acetate, ethyl acetate, toluene and chloroform, and evaporation of the solvent, and the obtained crude product can be purified by silica gel chromatography, recrystallization (n-hexane, ethyl acetate and the like), vacuum distillation, or the like, as required.

Further, after performing the asymmetric hydrolysis reaction, optically pure unreacted ester compound represented by the general formula (I) (optically active α-hydroxycarboxylic acid ester), which is not hydrolyzed and remains in the reaction mixture, exists in the reaction mixture in addition to the optically active α-hydroxycarboxylic acid represented by the general formula (I). By separating and purifying this optically active α-hydroxycarboxylic acid ester and then hydrolyzing the ester group, the optically active α-hydroxycarboxylic acid represented by the general formula (I) can be produced. The acid or base used for the hydrolysis is not particularly limited, so long as those added as an acid or a base in usual reactions are chosen. Examples include, for example, mineral acids such as hydrochloric acid and sulfuric acid, and bases such as sodium hydroxide, potassium hydroxide, sodium carbonate, potassium carbonate, sodium hydrogencarbonate, sodium hydride, lithium hydride and aqueous ammonia, and sodium hydroxide or potassium hydroxide can be preferably used. In the case of the hydrolysis with a base, it is desirable to choose appropriate conditions so that inversion of stereochemistry of the compound represented by the general formula (I) does not occur. The reaction solvent for the hydrolysis is not particularly limited, so long as a solvent is used which does not inhibit advance of the reaction and can fully dissolve the starting material. Examples include, for example, alcohols (methanol, ethanol and the like), dimethylformamide (DMF), N,N-dimethylacetamide (DMAc), diethyl ether, tetrahydrofuran, dioxane, water, acetone, mixtures of these, and the like, and alcohols, water and a mixture of these solvents can be preferably used. The reaction temperature is usually -20 to 150°C, preferably 10 to 30°C. Although the reaction time changes depending on the starting material, solvent, reaction temperature and the like to be applied, the period of time is usually 5 minutes to 36 hours, preferably 0 minutes to 16 hours.
The substrate can be produced by an ordinary method from an α-hydroxycarboxylic acid and an alcohol, which are not optically pure and cheaply supplied.

### Examples

The present invention will be explained more specifically with reference to examples. However, the scope of the present invention is not limited by these

### examples.

### Example 1

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid methyl ester (50 µL of 20% ethanol solution) as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.92 mg/mL of 2-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (R)-2-chloromandelic acid.
The reaction was performed in the same manner as that described above with each of the microorganisms listed in Table 1 instead of the aforementioned microorganism, and the results shown in the table were obtained.

**[Table 1]**

| | | | | | | |
|---|---|---|---|---|---|---|
| 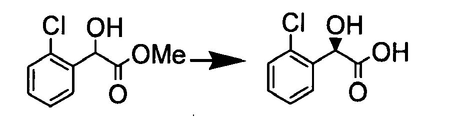 | | | | | | |
| (In the formula, Me represents methyl group, and the same shall following descriptions.) apply in the | | | | | | |

| Name of Microorganism | | Microorganism No. | | Production Amount | Absolute Configuration | Optical Purity |
|---|---|---|---|---|---|---|
| Genus | Species | Depository | No. | (mg/mL) | | (% e.e.) |
| *Leifsonia* | *aquatica* | JCM | 1368 | 2.63 | R | 88.0 |
| *Cylindrocarpon* | sp. | NBRC | 31855 | 3.23 | R | 100.0 |
| *Verticillium* | *leptobactrum* | IAM | 14729 | 1.02 | R | 61.1 |
| *Mycobacterium* | *smegmatis* | NBRC | 3154 | 1.18 | R | 100.0 |
| *Mycobacterium* | *phlei* | NBRC | 3158 | 0.81 | R | 100.0 |
| *Mycobacterium* | *vaccae* | NBRC | 14118 | 0.39 | R | 100.0 |
| *Rhodococcus* | *Equi* | JCM | 1313 | 0.32 | R | 100.0 |
| *Rhodococcus* | *fascians* | NBRC | 12155 | 0.39 | R | 100.0 |
| *Rhodococcus* | *wratislaviensis* | JCM | 9689 | 1.46 | R | 66.4 |
| *Exophiala* | *jeanselmei* | NBRC | 6857 | 0.70 | R | 100.0 |
| *Exophiala* | *dermatitidis* | NBRC | 8193 | 1.24 | R | 100.0 |
| *Rhinocladiella* | *ellisii* | NBRC | 101151 | 1.77 | R | 93 |
| *Rhinocladiella* | *atrovirens* | NBRC | 32362 | 2.68 | R | 88.2 |

### Example 2

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid methyl ester (50 µL of 20% ethanol solution) as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.71 mg/mL of 2-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (S)-2-chloromandelic acid.
The reaction was performed in the same manner as that described above with each of the microorganisms mentioned in Table 2 instead of the aforementioned microorganism, and the results shown in the table were obtained.

**Table 2]**

| | | | | | | |
|---|---|---|---|---|---|---|
| 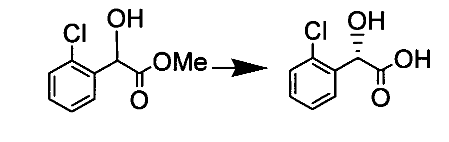 | | | | | | |
| Name of Microorganism | | Microorganism No. | | Production | Absolute Configuration | Optical Purity (% e.e.) |
| Genus | Species | Depos itory | No. | Amount (mg/mL) | | |
| *Rhodotorula* | *aurantiaca* | NBRC | 0951 | 0.93 | S | 60.0 |
| *Bacillus* | *cereus* | NBRC | 13690 | 1.50 | S | 99.2 |
| *Bacillus* | *cereus* | NBRC | 15305 | 0.94 | S | 100.0 |
| *Bacillus* | *cereus* | NBRC | 3514 | 1.19 | S | 100.0 |
| *Bacillus* | *fusiformis* | NBRC | 3528** | 0.99 | S | 100.0 |
| *Brevundimonas* | *diminuta* | NBRC | 14213 | 2.35 | S | 65.0 |
| *Brevundimonas* | *diminuta* | NBRC | 12697 | 1.46 | S | 65.9 |
| *Brevundimonas* | *diminuta* | JCM | 2789 | 2.05 | S | 60.0 |
| *Pseudomonas* | *aeruginosa* | NBRC | 3918 | 1.37 | S | 63.1 |
| *Rhizobium* | *radiobacter* | NBRC | 13263 | 5.37 | S | 94.9 |
| *Aspergillus* | *ochraceus* | JCM | 1958 | 0.30 | S | 100.0 |
| *Aspergillus* | *oryzae* | IAM | 2630 | 0.33 | S | 100.0 |
| *Beauveria* | *bassiana* | NBRC | 4848 | 0.64 | S | 100.0 |
| *Penicillium* | *spinulosum* | IAM | 7047 | 0.27 | S | 100.0 |
| *Nocardia* | *asteroides* | NBRC | 3384 | 0.31 | S | 100.0 |
| *Nocardia* | *asteroides* | NBRC | 3424 | 0.51 | S | 100.0 |
| *Nocardia* | *globerula* | NBRC | 13510 | 0.37 | S | 100.0 |
| *Gordonia* | *bronchialis* | JCM | 3198 | 0.32 | S | 100.0 |
| *Gordonia* | *sputi* | JCM | 6047 | 0.30 | S | 100.0 |
| *Rhodococcus* | *erythropolis* | JCM | 6826 | 1.18 | S | 63.8 |
| *Gordonia* | *rubripertincta* | JCM | 3199 | 0.58 | S | 63.0 |
| *Rhodococcus* | sp. | NBRC | 13162 | 0.49 | S | 100.0 |
| *Gordonia* | *sputi* | JCM | 3228 | 0.30 | S | 100.0 |
| *Rhodococcus* | *rhodochrous* | ATCC | 12674 | 2.13 | S | 68.9 |
| *Rhodococcus* | *erythropolis* | IAM | 1414 | 0.35 | S | 100.0 |
| *Ramichloridium* | *anceps* | NBRC | 9448 | 0.87 | S | 100 |
| *Porphyrobacter* | *sanguineus* | NBRC | 15763 | 0.65 | S | 64 |

### Example 3

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid methyl ester (50 µ L of 20% ethanol solution) as racemate, and they were mixed to obtain 1 mL of a reaction mixture and reacted at 30°C for 72 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 5.04 mg/mL of 2-chloromandelic acid methyl ester remained. In order to determine optical purity of the remained substance, a sample was subjected to HPLC analysis (CHIRALCEL OJ, DAICEL, diameter: 4.6 mm, length: 250 mm, eluent: n-hexane/IPA = 9/1, flow rate: 1.0 mL/minute, detection wavelength: UV 254 nm). As a result, it was found that the product was (R)-2-chloromandelic acid methyl ester.
The reaction was performed in the same manner as that described above with the microorganism mentioned in Table 3 instead of the aforementioned microorganism, and the results shown in the table were obtained.

**[Table 3]**

| | | | | | | |
|---|---|---|---|---|---|---|
| 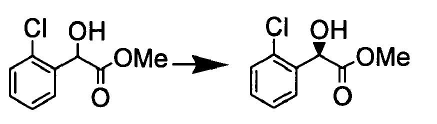 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Rhizobium* | *radiobacter* | NBRC | 13263 | 5.04 | R | 98.2 |
| *Bacillus* | *cereus* | NBRC | 15305 | 5.81 | R | 45.5 |

### Example 4

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µ L of 1 M MES buffer (pH 6.5) and 10 mg of 2-chloromandelic acid methyl ester (50 µ L of 20% ethanol solution) as racemate, and they were mixed to obtain 1 mL of a reaction mixture and reacted at 30°C for 72 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 4.27 mg/mL of unreacted 2-chloromandelic acid methyl ester remained. In order to determine optical purity of the remained substance, a sample was subjected to HPLC analysis (CHIRALCEL OJ, DAICEL, diameter: 4.6 mm, length: 250 mm, eluent: n-hexane/IPA = 9/1, flow rate: 1.0 mL/minute, detection wavelength: UV 254 nm). As a result, it was found that the product was (S)-2-chloromandelic acid methyl ester.
The reaction was performed in the same manner as that described above with the microorganism mentioned in Table 4 instead of the aforementioned microorganism, and the results shown in the table were obtained.

[Table 4]

| | | | | | | |
|---|---|---|---|---|---|---|
| 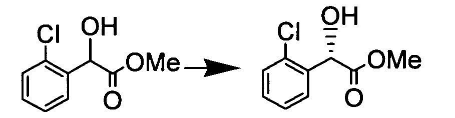 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Exophiala* | *dermatitidis* | NBRC | 8193 | 4.27 | S | 96.7 |
| *Mycobacterium* | *smegmatis* | NBRC | 3154 | 4.39 | S | 81.3 |

### Example 5-1

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 4-chloromandelic acid methyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 2.25 mg/mL of 4-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (R)-4-chloromandelic acid.

**[Table 5]**

| | | | | | | |
|---|---|---|---|---|---|---|
| 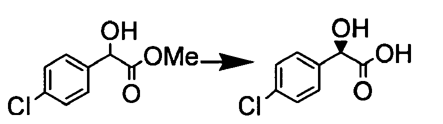 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Exophiala* | *dermatitidis* | NBRC | 8193 | 2.25 | R | 64.6 |

### Example 5-2

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 4-trifluoromethylmandelic acid methyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 2.62 mg/mL of 4-trifluoromethylmandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (R)-4-trifluoromethyl-chloromandelic acid.

**[Table 6]**

| | | | | | | |
|---|---|---|---|---|---|---|
| 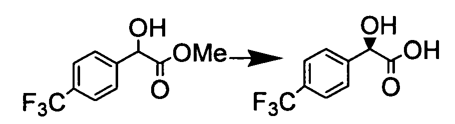 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Exophiala* | *dermatitidis* | NBRC | 8193 | 2.62 | R | 53.2 |

### Example 5-3

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 4-methoxymandelic acid methyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 2.01 mg/mL of 4-methoxymandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (R)-4-methoxymandelic acid.
The reaction was performed in the same manner as that described above with the microorganism mentioned in Table 7 instead of the aforementioned strain, and the results shown in the table were obtained.

**[Table 7]**

| | | | | | | |
|---|---|---|---|---|---|---|
| 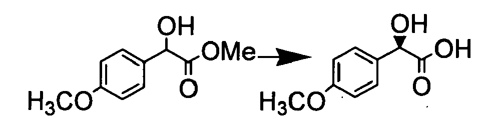 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Exophiala* | *dermatitidis* | NBRC | 8193 | 2.01 | R | 77.7 |

### Example 5-4

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid ethyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 2.03 mg/mL of 2-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (R)-2-chloromandelic acid.

**[Table 8]**

| | | | | | | |
|---|---|---|---|---|---|---|
| 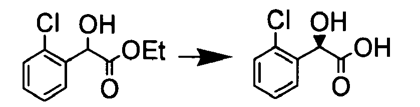 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Exophiala* | *dermatitidis* | NBRC | 8193 | 2.03 | R | 100 |

### Example 5-5

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50µL of 1 M MES buffer (pH 6.5) and 10 mg of 2-chloromandelic acid isopropyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.53 mg/mL of 2-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (R)-2-chloromandelic acid.
The reaction was performed in the same manner as that described above with each of the microorganisms mentioned in Table 9 instead of the aforementioned strain, and the results shown in the table were obtained.

**[Table 9]**

| | | | | | | |
|---|---|---|---|---|---|---|
| 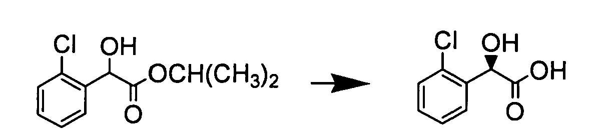 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Exophiala* | *dermatitidis* | NBRC | 8193 | 1.53 | R | 100 |
| *Mycobacterium* | *smegmatis* | NBRC | 3154 | 0.02 | R | 100.0 |
| *Cylindrocarpon* | sp. | NBRC | 31855 | 0.05 | R | 100.0 |

### Example 5-6

The *Exophiala dermatitidis* NBRC 8193 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid benzyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.42 mg/mL of 2-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (R)-2-chloromandelic acid.
The reaction was performed in the same manner as that described above with the microorganism mentioned in Table 10 instead of the aforementioned strain, and the results shown in Table 10 were obtained.

**[Table 10]**

| | | | | | | |
|---|---|---|---|---|---|---|
| 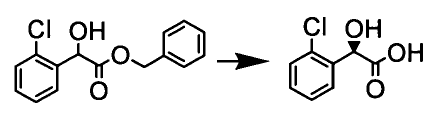 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Exophiala* | *dermatitidis* | NBRC | 8193 | 1.42 | R | 83.8 |
| *Cylindrocarpon* | sp. | NBRC | 31855 | 0.53 | R | 54.1 |

### Example 5-7

The *Exophiala jeanselmei* NBRC 6857 strain was cultured in a medium containing 1% of glucose, 0.5% of peptone and 0.3% of yeast extract at 25°C for 3 days with shaking. After the culture, cell bodies were obtained by centrifuging 0.28 mL of the culture medium. To these cell bodies were added an appropriate volume of water, 100 µL of 0.5 M MES buffer (pH 6.5) and 5 mg of atrolactic acid methyl ester as racemate, and they were mixed to obtain 0.5 mL of a reaction mixture and reacted at 30°C for 30 minutes with shaking. After completion of the reaction, the reaction mixture was centrifuged or filtered, and the supernatant was subjected to HPLC analysis (Inertsil ODS-3, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 0.11 mg/mL of atrolactic acid was produced. In order to determine optical purity of the product, a sample was subjected to HPLC analysis (GEL PACKED COLUMN CRS10W, MCI, diameter: 4.6 mm, length: 50 mm, eluent: 90% of 0.2 mM CuSO₄ and 10% of acetonitrile, flow rate: 2.0 mL/minute, detection wavelength: UV 254 nm). As a result, it was found that the product was (R)-atrolactic acid. The optical purity was 100%ee.

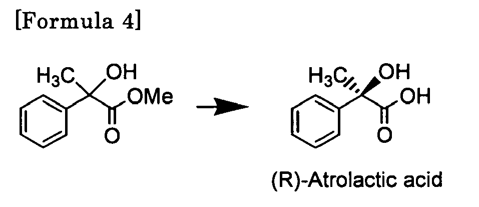

### Example 5-8

The *Exophiala jeanselmei* NBRC 6857 strain was cultured in 5 mL of a medium containing 1% of glucose, 0.5% of peptone and 0.3% of yeast extract at 25°C for 3 days with shaking. Cell bodies were obtained by centrifuging 0.28 mL of the culture medium. To these cell bodies were added an appropriate volume of water, 100 µL of 1 M MES buffer (pH 6.5) and 5 mg of 4-fluoromandelic acid methyl ester as racemate, and they were mixed to obtain 0.5 mL of a reaction mixture and reacted at 30°C for 30 minutes with shaking. After completion of the reaction, the reaction mixture was centrifuged, and the supernatant was subjected to HPLC analysis (Inertsil ODS-3, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 0.19 mg/mL of 4-fluoromandelic acid was produced. In order to determine optical purity of the product, a sample was subjected to HPLC analysis (GEL PACKED COLUMN CRS10W, MCI, diameter: 4.6 mm, length: 50 mm, eluent: 90% of 2 mM CuSO₄ and 10% of acetonitrile, flow rate: 1.0 mL/minute, detection wavelength: UV 254 nm). As a result, it was found that the product was (R)-4-fluoromandelic acid. The optical purity was 77%ee.

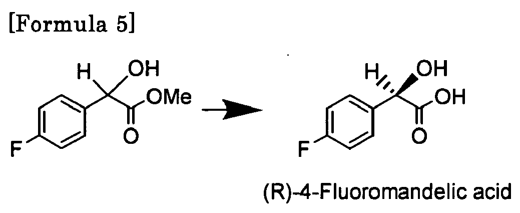

### Example 5-9

The *Exophiala jeanselmei* NBRC 6857 strain was cultured in 5 mL of a medium containing 1% of glucose, 0.5% of peptone and 0.3% of yeast extract at 25°C for 3 days with shaking. Cell bodies were obtained by centrifuging 0.28 mL of the culture medium. To these cell bodies were added an appropriate volume of water, 100 µ L of 0.5 M MES buffer (pH 6.5) and 5 mg of 3-chloromandelic acid methyl ester, and they were mixed to obtain 0.5 mL of a reaction mixture and reacted at 30°C for 30 minutes with shaking. After completion of the reaction, the reaction mixture was centrifuged, and the supernatant was subjected to HPLC analysis (Inertsil ODS-3, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 0.094 mg/mL of 3-chloromandelic acid was produced. In order to determine optical purity of the product, a sample was subjected to HPLC analysis (GEL PACKED COLUMN CRS10W, MCI, diameter: 4.6 mm, length: 50 mm, eluent: 90% of 0.2 mM CuSO₄ and 10% of acetonitrile, flow rate: 1.0 mL/minute, detection wavelength: UV 254 nm). As a result, it was found that the product was (R)-3-chloromandelic acid. The optical purity was 67.7%ee.

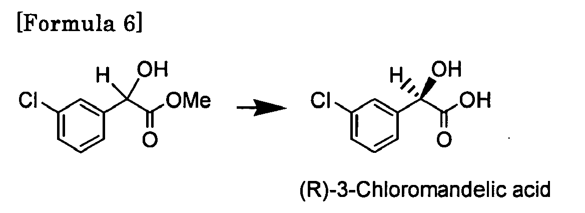

### Example 6-1

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 4-chloromandelic acid methyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.46 mg/mL of 4-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (S)-4-chloromandelic acid.

**[Table 11]**

| | | | | | | |
|---|---|---|---|---|---|---|
| 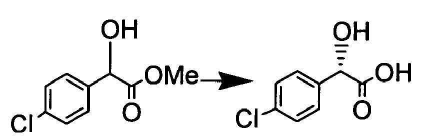 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Rhizobium* | *radiobacter* | NBRC | 13263 | 1.46 | S | 72.3 |

### Example 6-2

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µ L of 1 M MES buffer (pH 6.5) and 10 mg of 4-trifluoromethylmandelic acid methyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.21 mg/mL of 4-trifluoromethylmandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (S)-4-trifluoromethylmandelic acid.

**[Table 12]**

| | | | | | | |
|---|---|---|---|---|---|---|
| 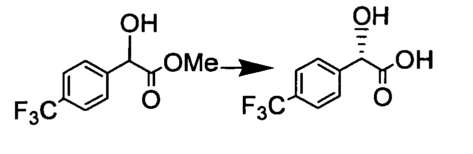 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Rhizobium* | *radiobacter* | NBRC | 13263 | 1.21 | S | 44.8 |

### Example 6-3

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 4-methoxymandelic acid methyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.08 mg/mL of 4-methoxymandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (S)-4-methoxymandelic acid.

**[Table 13]**

| | | | | | | |
|---|---|---|---|---|---|---|
| 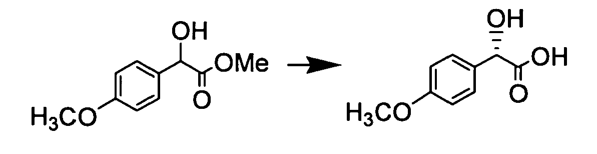 | | | | | | |
| Name of Microorganism | | Microorganism No. | | Production Amount (mg/mL) | Absolute Configuration | Optical Purity (% e.e.) |
| Genus | Species | Depository | No. | | | |
| *Rhizobium* | *radiobacter* | NBRC | 13263 | 1.08 | S | 43.1 |

### Example 6-4

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid ethyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.96 mg/mL of 2-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (S)-2-chloromandelic acid.

**[Table 14]**

| | | | | | | |
|---|---|---|---|---|---|---|
| 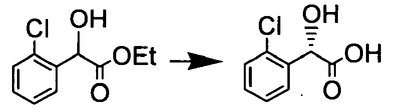 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Rhizobium* | *radiobacter* | NBRC | 13263 | 1.96 | S | 95.2 |

### Example 6-5

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid isopropyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.91 mg/mL of 2-chloromandelic acid was produced. In order to determine optical purity of the product, a sample was subjected to HPLC analysis (GEL PACKED COLUMN CRS10W, Mitsubishi Chemical, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (S)-2-chloromandelic acid.
The reaction was performed in the same manner as that described above with the microorganism mentioned in Table 15 instead of the aforementioned strain, and the results shown in the table were obtained.

**[Table 15]**

| | | | | | | |
|---|---|---|---|---|---|---|
| 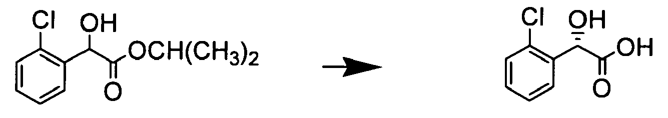 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Rhizobium* | *Radiobacter* | NBRC | 13263 | 1.91 | S | 100 |
| *Bacillus* | *Cereus* | NBRC | 3514 | 0.80 | S | 77.8 |

### Example 6-6

The *Rhizobium radiobacter* NBRC 13263 strain was cultured in 5 mL of a medium containing 1% of glucose, 0.5% of peptone and 0.3% of yeast extract at 25°C for 6 days with shaking. Cell bodies were obtained by centrifugation or filtration. To these cell bodies were added an appropriate volume of water, 50 µL of 1 M MES buffer (pH 6.5) and 10 mg of 2-chloromandelic acid benzyl ester as racemate, and they were mixed to obtain 1 mL of a reaction mixture and reacted at 30°C for 20 hours with shaking. After completion of the reaction, the reaction mixture was centrifuged or filtered, and the supernatant was subjected to HPLC analysis (Inertsil ODS-3, GL Science, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 1.62 mg/mL of 2-chloromandelic acid was produced. In order to determine optical purity of the product, a sample was subjected to HPLC analysis (GEL PACKED COLUMN CRS10W, MCI, diameter: 4.6 mm, length: 50 mm, eluent: 85% of 0.2 mM CuSO₄ and 15% of acetonitrile, flow rate: 2.0 mL/minute, detection wavelength: UV 254 nm). As a result, it was found that the product was (S)-2-chloromandelic acid.
The reaction was performed in the same manner as that described above with each of the microorganisms mentioned in Table 16 instead of the aforementioned strain, and the results shown in the table were obtained.

**[Table 16]**

| | | | | | | |
|---|---|---|---|---|---|---|
| 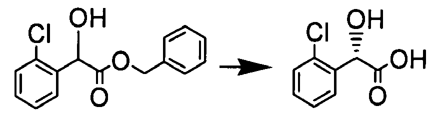 | | | | | | |

| Name of Microorganism | | Microorganism No. | | Production Amount (mg/mL) | Absolute Configuration | Optical Purity (% e.e.) |
|---|---|---|---|---|---|---|
| Genus | Species | Depository | No. | | | |
| *Rhizobium* | *radiobacter* | NBRC | 13263 | 1.62 | S | 90.6 |
| *Bacillus* | *cereus* | NBRC | 15305 | 0.03 | S | 100.0 |
| *Bacillus* | *cereus* | NBRC | 3514 | 0.65 | S | 70.6 |

### Example 6-7

The *Rhizobium radiobacter* NBRC 13263 strain was cultured in a medium containing 1% of glucose, 0.5% of peptone and 0.3% of yeast extract at 25°C for 3 days with shaking. After the culture, cell bodies were obtained by centrifuging 9.1 mL of the culture medium. To these cell bodies were added an appropriate volume of water, 100 µ L of 0.5 M MES buffer (pH 6.5) and 5 mg of atrolactic acid methyl ester, and they were mixed to obtain 0.5 mL of a reaction mixture and reacted at 30°C for 30 minutes with shaking. After completion of the reaction, the reaction mixture was centrifuged, and the supernatant was subjected to HPLC analysis (Inertsil ODS-3, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 0.15 mg/mL of atrolactic acid was produced. In order to determine optical purity of the product, a sample was subjected to HPLC analysis (GEL PACKED COLUMN CRS10W, MCI, diameter: 4.6 mm, length: 50 mm, eluent: 90% of 2 mM CuSO₄ and 10% of acetonitrile, flow rate: 1.0 mL/minute, detection wavelength: UV 254 nm). As a result, it was found that the product was (S)-2-atrolactic acid. The optical purity was 55%ee.

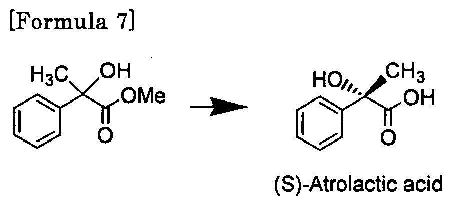

### Example 6-8

The *Rhizobium radiobacter* NBRC 13263 strain was cultured in a medium containing 1% of glucose, 0.5% of peptone and 0.3% of yeast extract at 25°C for 3 days with shaking. After the culture, cell bodies were obtained by centrifuging 9.1 mL of the culture medium. To these cell bodies were added an appropriate volume of water, 100 µ L of 0.5 M MES buffer (pH 6.5) and 5 mg of 4-fluoromandelic acid methyl ester, and they were mixed to obtain 0.5 mL of a reaction mixture and reacted at 30°C for 30 minutes with shaking. After completion of the reaction, the reaction mixture was centrifuged, and the supernatant was subjected to HPLC analysis (Inertsil ODS-3, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 0.22 mg/mL of 4-fluoromandelic acid was produced. In order to determine optical purity of the product, a sample was subjected to HPLC analysis (GEL PACKED COLUMN CRS10W, MCI, diameter: 4.6 mm, length: 50 mm, eluent: 90% of 2 mM CuSO₄ and 10% of acetonitrile, flow rate: 1.0 mL/minute, detection wavelength: UV 254 nm). As a result, it was found that the product was (S)-4-fluoromandelic acid. The optical purity was 100%ee.

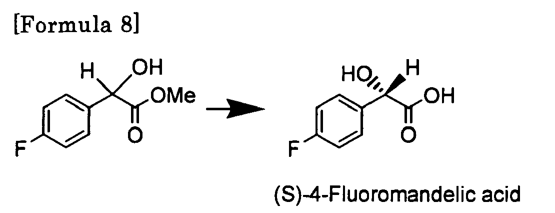

### Example 6-9

The *Rhizobium radiobacter* NBRC 13263 strain was cultured in a medium containing 1% of glucose, 0.5% of peptone and 0.3% of yeast extract at 25°C for 3 days with shaking. After the culture, cell bodies were obtained by centrifuging 9.1 mL of the culture medium. To these cell bodies were added an appropriate volume of water, 100 µ L of 0.5 M MES buffer (pH 6.5) and 5 mg of 3-chloromandelic acid methyl ester, and they were mixed to obtain a volume of 0.5 mL and reacted at 30°C for 30 minutes with shaking. After completion of the reaction, the reaction mixture was centrifuged, and the supernatant was subjected to HPLC analysis (Inertsil ODS-3, diameter: 4.6 mm, length: 75 mm, eluent: 25% of acetonitrile and 75% of 0.05 M sodium phosphate buffer, pH 2.5, flow rate: 1.0 mL/minute, detection wavelength: UV 254 nm). As a result, it was found that 0.23 mg/mL of 3-chloromandelic acid was produced. In order to determine optical purity of the product, a sample was subjected to HPLC analysis (GEL PACKED COLUMN CRS10W, MCI, diameter: 4.6 mm, length: 50 mm, eluent: 90% of 2 mM CuSO₄ and 10% of acetonitrile, flow rate: 1.0 mL/minute, detection wavelength: UV 254 nm). As a result, it was found that the product was (S)-3-chloromandelic acid. The optical purity was 76.9%ee.

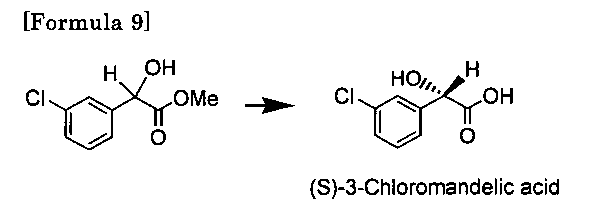

### Industrial Applicability

Optically active α-hydroxycarboxylic acids or optically active α-hydroxycarboxylic acid esters having an extremely high optical purity can be conveniently produced at a low cost by the method of the present invention. Accordingly, the method of the present invention is useful for industrial production of optically active α-hydroxycarboxylic acids or optically active α-hydroxycarboxylic acid esters. By using optically active α-hydroxycarboxylic acids or ester derivatives thereof produced by the method of the present invention, various biologically active compounds can be produced.

## Claims

1. A method for producing a compound represented by the general formula (I): 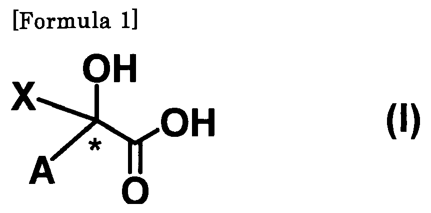 [wherein A represents a residue of a 5- or 6-membered cyclic compound, wherein the cyclic compound is selected from an aromatic compound, a partially saturated cyclic compound, or a saturated cyclic compound, and wherein said compound may have one or more heteroatoms as a ring-constituting atom, and may have a substituent on the ring (the substituent consists of one substituent or two or more substituents selected from the group consisting of a halogen atom, an alkyl group having 1 to 4 carbon atoms which may have a substituent, an alkyloxy group having 1 to 4 carbon atoms which may have a substituent, a hydroxyl group which may be protected with a protective group, an amino group which may be protected with a protective group, and nitro group, and when two or more substituents exist, they may be the same or different, and they may bind to each other to form a ring), X represents hydrogen atom or an alkyl group having 1 to 4 carbon atoms, and * indicates a carbon atom in the S- or R-configuration], which comprises the step of treating a compound represented by following general formula (II): 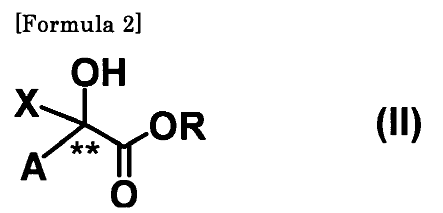 [wherein A and X have the same meanings as those defined above, and R represents an alkyl group having 1 to 4 carbon atoms (the alkyl group may be substituted with an aryl group), provided that the compound represented by the general formula (II) is not optically pure with reference to the carbon atom indicated with **] with cell bodies or a culture, or a processed product or an extract thereof of a microorganism selected from the group consisting of microorganisms belonging to the genus *Leifsonia,* genus *Cylindrocarpon,* genus *Verticillium,* genus *Mycobacterium,* genus *Rhodococcus,* genus *Exophiala,* genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhinocladiella,* genus *Ramichloridium* or genus *Porphyrobacter.*

2. The method according to claim 1, wherein A is chlorophenyl group, R is an alkyl group having 1 to 4 carbon atoms or benzyl group, and X is hydrogen atom.

3. The method according to claim 1, wherein A is o-chlorophenyl group, R is methyl group, and X is hydrogen atom.

4. The method according to any one of claims 1 to 3, wherein the microorganism is a microorganism selected from the group consisting of *Leifsonia aquatica, Cylindrocarpon* sp., *Verticillium leptobactrum, Mycobacterium smegmatis, Mycobacterium phlei, Mycobacterium vaccae, Rhodococcus equi, Rhodococcus fascians, Rhodococcus wratislaviensis, Exophiala jeanselmei, Exophiala dermatitidis, Rhodotorula aurantiaca, Bacillus cereus, Bacillus fusiformis, Brevundimonas diminuta, Pseudomonas aeruginosa, Rhizobium radiobacter, Aspergillus ochraceus, Aspergillus oryzae, Beauveria bassiana, Penicillium spinulosum, Nocardia asteroides, Nocardia globerula, Gordonia bronchialis, Gordonia sputi, Gordonia rubripertincta, Rhodococcus* sp., *Rhodococcus rhodochrous, Rhodococcus erythropolis, Rhinocladiella ellisii, Rhinocladiella atrovirens, Ramichloridium anceps,* and *Porphyrobacter sanguineus.*

5. The method according to any one of claims 1 to 3, which comprises the step of hydrolyzing the ester group of the compound represented by the general formula (II) wherein the carbon atom indicated with ** is in the R-configuration to obtain the compound represented by the general formula (I) wherein the carbon atom indicated with * is in the R-configuration, and wherein the microorganism is a microorganism selected from the group consisting of microorganisms belonging to the genus *Leifsonia,* genus *Cylindrocarpon,* genus *Verticillium,* genus *Mycobacterium,* genus *Rhodococcus,* genus *Exophiala,* or genus *Rhinocladiella*.

6. The method according to claim 5, wherein the microorganism is a microorganism selected from the group consisting of *Leifsonia aquatica, Cylindrocarpon* sp., *Verticillium leptobactrum, Mycobacterium smegmatis, Mycobacterium phlei, Mycobacterium vaccae, Rhodococcus equi, Rhodococcus fascians, Rhodococcus wratislaviensis, Exophiala jeanselmei, Exophiala dermatitidis, Rhinocladiella ellisii,* and *Rhinocladiella atrovirens.*

7. The method according to any one of claims 1 to 3, which comprises the step of hydrolyzing the ester group of the compound represented by the general formula (II) where the carbon atom indicated with ** is in the S-configuration to obtain the compound represented by the general formula (I) wherein the carbon atom indicated with * is in the S-configuration, and wherein the microorganism is a microorganism selected from the group consisting of microorganisms belonging to the genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhodococcus,* genus *Ramichloridium,* or genus *Porphyrobacter.*

8. The method according to claim 7, wherein the microorganism is a microorganism selected from the group consisting of *Rhodotorula aurantiaca, Bacillus cereus, Bacillus fusiformis, Brevundimonas diminuta, Pseudomonas aeruginosa, Rhizobium radiobacter, Aspergillus ochraceus, Aspergillus oryzae, Beauveria bassiana, Penicillium spinulosum, Nocardia asteroides, Nocardia globerula, Gordonia bronchialis, Gordonia sputi, Gordonia rubripertincta, Rhodococcus* sp., *Rhodococcus rhodochrous, Rhodococcus erythropolis, Ramichloridium anceps,* and *Porphyrobacter sanguineus.*

9. A method for producing a compound represented by the following general formula (III): 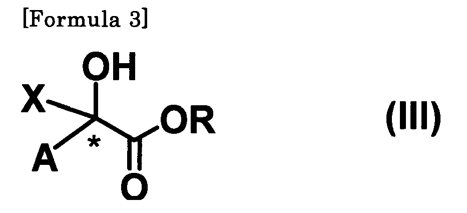 (wherein A, R, X and * have the same meanings as those defied above), which comprises the step of treating a compound represented by the aforementioned general formula (II) (wherein A, R, X, and ** have the same meanings as those defined above) with cell bodies or a culture, or a processed product or an extract thereof of a microorganism selected from the group consisting of microorganisms belonging to the genus *Leifsonia,* genus *Cylindrocarpon,* genus *Verticillium,* genus *Mycobacterium,* genus *Rhodococcus,* genus *Exophiala,* genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhinocladiella* group, genus *Ramichloridium* or genus *Porphyrobacter to* hydrolyze the ester group of the compound represented by the general formula (II) wherein the carbon atom indicated with ** is in the S- or R-configuration, and separating the unreacted ester compound which is optically pure and remains in a reaction mixture.

10. The method according to claim 9, wherein A is chlorophenyl group, R is an alkyl group having 1 to 4 carbon atoms or benzyl group, and X is hydrogen atom.

11. The method according to claim 9, wherein A is o-chlorophenyl group, R is methyl group, and X is hydrogen atom.

12. The method according to claim any one of claims 9 to 11, wherein the microorganism is a microorganism selected from the group consisting of *Leifsonia aquatica, Cylindrocarpon* sp., *Verticillium leptobactrum, Mycobacterium smegmatis, Mycobacterium phlei, Mycobacterium vaccae, Rhodococcus equi, Rhodococcus fascians, Rhodococcus wratislaviensis, Exophiala jeanselmei, Exophiala dermatitidis, Rhodotorula aurantiaca, Bacillus cereus, Bacillus fusiformis, Brevundimonas diminuta, Pseudomonas aeruginosa, Rhizobium radiobacter, Aspergillus ochraceus, Aspergillus oryzae, Beauveria bassiana, Penicillium spinulosum, Nocardia asteroides, Nocardia globerula, Gordonia bronchialis, Gordonia sputi, Gordonia rubripertincta, Rhodococcus erythropolis, Rhodococcus* sp., *Rhodococcus rhodochrous, Rhodococcus erythropolis, Rhinocladiella ellisii, Rhinocladiella atrovirens, Ramichloridium anceps,* and *Porphyrobacter sanguineus.*

13. The method according to any one of claims 9 to 11, which comprises the step of hydrolyzing the ester group of the compound represented by the general formula (II) wherein the carbon atom indicated with ** is in the S-configuration, and wherein the microorganism is a microorganism selected from the group consisting of microorganisms belonging to the genus *Rhodotorula,* genus *Bacillus,* genus *Brevundimonas,* genus *Pseudomonas,* genus *Rhizobium,* genus *Aspergillus,* genus *Beauveria,* genus *Penicillium,* genus *Nocardia,* genus *Gordonia,* genus *Rhodococcus,* genus *Ramichloridium,* or genus *Porphyrobacter.*

14. The method according to claim 13, wherein the microorganism is a microorganism selected from the group consisting of *Rhodotorula aurantiaca, Bacillus cereus, Bacillus fusiformis, Brevundimonas diminuta, Pseudomonas aeruginosa, Rhizobium radiobacter, Aspergillus ochraceus, Aspergillus oryzae, Bea uveria bassiana, Penicillium spinulosum, Nocardia asteroides, Nocardia globerula, Gordonia bronchialis, Gordonia sputi, Gordonia rubripertincta, Rhodococcus* sp., *Rhodococcus rhodochrous, Rhodococcus erythropolis, Ramichloridium anceps,* and *Porphyrobacter sanguineus.*

15. The method according to any one of claims 9 to 11, which comprises the step of hydrolyzing the ester group of the compound represented by the general formula (II) wherein the carbon atom indicated with ** is in the R-configuration, and wherein the microorganism is a microorganism selected from the group consisting of microorganisms belonging to the genus *Leifsonia,* genus *Cylindrocarpon,* genus *Verticillium,* genus *Mycobacterium,* genus *Rhodococcus,* genus *Exophiala* or genus *Rhinocladiella.*

16. The method according to claim 15, wherein the microorganism is a microorganism selected from the group consisting of *Leifsonia aquatica, Cylindrocarpon* sp., *Verticillium leptobactrum, Mycobacterium smegmatis, Mycobacterium phlei, Mycobacterium vaccae, Rhodococcus equi, Rhodococcus fascians, Rhodococcus wratislaviensis, Exophiala jeanselmei, Exophiala dermatitidis, Rhinocladiella ellisii,* and *Rhinocladiella atrovirens.*

17. Clopidogrel produced by using an optically active compound obtained by the method according to any one of claims 1 to 3 and claims 9 to 11, or a method for producing the same.
